# EUROPEAN PATENT APPLICATION

(11) **EP 3 836 008 A1**
(43) Date of publication of application: **16.06.2021**
(21) Application number: 20159579.0
(22) Date of filing: 26.02.2020
(51) Int. Cl.: G06K 9/00

(54) **METHOD FOR AUTOMATICALLY MARKING MUSCLE FEATURE POINTS ON FACE**

(30) Priority: 09.12.2019 CN 201911251805
(71) Applicant: CAL-COMP BIG DATA, INC, Shenkeng, New Taipei City 222 (TW)
(72) Inventor: WANG, Hong-Chun, 222 NEW TAIPEI CITY (TW)
(74) Representative: 2K Patentanwälte Blasberg Kewitz & Reichel

(57) **Abstract**

A method for automatically marking muscle feature points on face implemented by a face image analysis apparatus (1) includes following steps: obtaining a to-be-identified image (2) showing a face of a user; performing a face recognition procedure to the to-be-identified image (2) for obtaining multiple strong reference points on the face; performing a fuzzy comparison procedure on the face of the to-be-identified image (2) based on a pre-trained training model (153) for generating a comparison result; automatically marking multiple muscle feature points (3) on the face according to the comparison result, wherein the multiple muscle feature points (3) respectively locate at multiple weak reference points of the face; and, displaying the multiple muscle feature points (3) and the to-be-identified image (2) on a display unit (11).

## Description

### BACKGROUND OF THE INVENTION

### 1. Technical Field

The invention relates to a marking method of a face, and specifically to a marking method for marking muscle feature points on a face.

### 2. Description of Related Art

Human's muscle (especially face muscle) will slowly slacken and droop while people age, and some users choose to use care products to maintain their muscle and skin, use cosmetics to cover the slackened muscle, or work out for slowing down the speed of muscle slackening.

General speaking, users will be sitting in front of the mirror for using the care products and/or the cosmetics, or using the care products and/or the cosmetics through the assistance of smart phones, laptops or special makeup assisting devices for improving the speed and the quality of using the same.

However, the above devices can only assist the users in using the care products/cosmetics, but it cannot actively analyze user's muscle status. Therefore, users cannot be aware of whether the care products/cosmetics are working after using it for a period of time.

According to the above problem, a newly method should be provied in the field for effectively analyzing user's facial image and automatically marking a plurality of muscle feature points on the facial image for indicating user's current muscle status, so the user can easily realize his/her current muscle status and determines whether the currently applied care products/cosmetics are effective.

### SUMMARY OF THE INVENTION

The invention is directed to a method for automatically marking muscle feature points on a face, which can automatically mark a plurality of muscle feature points on user's facial image for indicating user's current facial muscle status.

In one of the exemplary embodiments, the method of the present invention is applied to a facial image analyzing device and includes following steps of: obtaining a to-be-identified image, wherein the to-be-identified image at least includes a face of a user; performing a face recognition procedure to the to-be-identified image for obtaining multiple strong reference points on the face; performing a fuzzy comparison procedure on the to-be-identified image based on a pre-trained training model for generating a comparison result; automatically marking multiple muscle feature points on the to-be-identified image according to the comparison result, wherein the multiple muscle feature points respectively locate at multiple weak reference points of the face; and, displaying the to-be-identified image and the multiple muscle feature points overlapped on the to-be-identified image by a display unit.

In comparison with related art, the present invention can identify user's facial image and automatically mark at least four muscle feature points on user's face, where the marked muscle feature points can be used to indicate user's facial muscle status. Therefore, user can be quickly aware of his/her current facial muscle status through the marked and displayed muscle feature points, so as to check whether the care products/cosmetics currently used are effective or not.

### BRIEF DESCRIPTION OF THE DRAWINGS

FIG. 1 is a schematic diagram of a facial image analyzing device according to a first embodiment of the present invention.
FIG. 2 is a block diagram of the facial image analyzing device according to a first embodiment of the present invention.
FIG. 3 is a schematic diagram showing muscle feature points according to a first embodiment of the present invention.
FIG. 4 is a flowchart for establishing a training model according to a first embodiment of the present invention.
FIG. 5 is a schematic diagram showing the marked location of the muscle feature points according to a first embodiment of the present invention.
FIG. 6 is a schematic diagram showing training data according to a first embodiment of the present invention.
FIG. 7 is an identification flowchart according to a first embodiment of the present invention.
FIG. 8 is an identification flowchart according to a second embodiment of the present invention.
FIG. 9 is a schematic diagram showing a training model according to a first embodiment of the present invention.
FIG. 10 is a schematic diagram showing the adjustment of the feature points according to a first embodiment of the present invention.

### DETAILED DESCRIPTION OF THE INVENTION

The invention is directed to a method for automatically marking muscle feature points on a face, which can automatically mark a plurality of muscle feature points on user's facial image for indicating user's current facial muscle status.

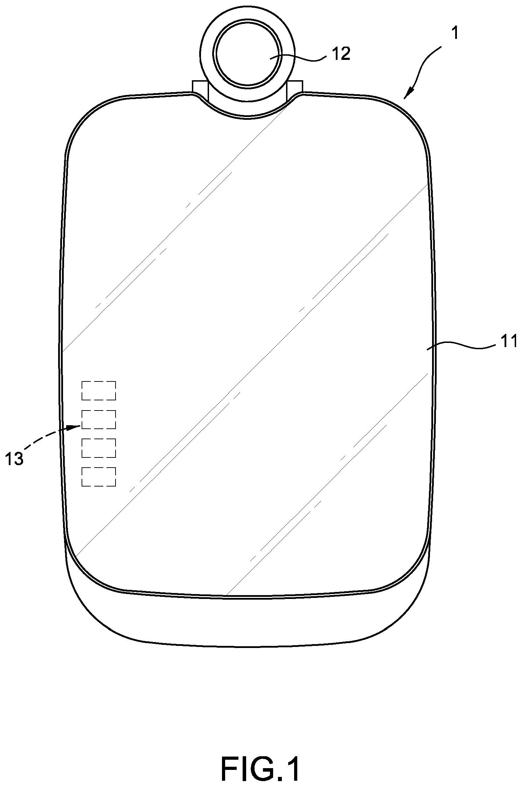

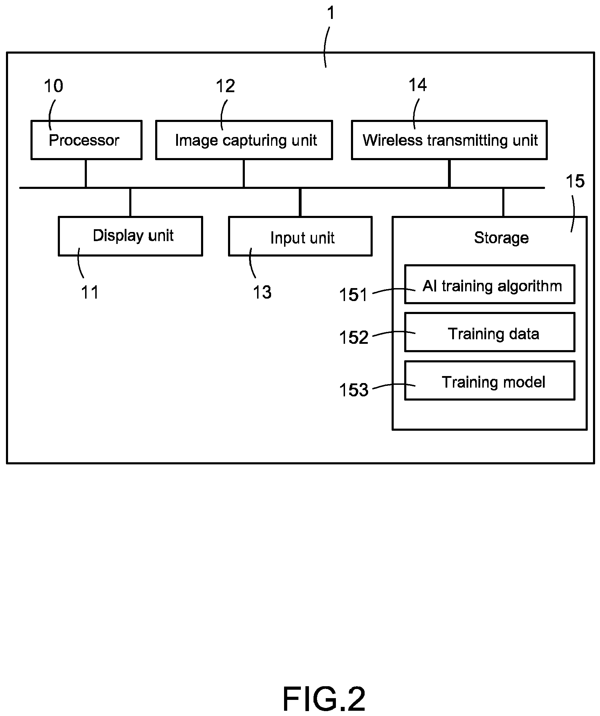

Please refer to FIG. 1 and FIG. 2, wherein FIG. 1 is a schematic diagram of a facial image analyzing device according to a first embodiment of the present invention, and FIG. 2 is a block diagram of the facial image analyzing device according to a first embodiment of the present invention.

A method for automatically marking muscle feature points on face is disclosed by the present invention (referred to as the marking method hereinafter), and the marking method is mainly applied to a facial image analyzing device 1 (referred to as the analyzing device 1 hereinafter) as disclosed in FIG. 1 and FIG. 2. Specifically, one of the technical features of the present invention is to obtain user's facial image through the analyzing device 1, then identifies the facial image based on a pre-trained Artificial Intelligent (AI) model, and then marks multiple muscle feature points on the facial image for indicating user's current facial muscle status according to an identification result. By automatically marking the multiple muscle feature points through the analyzing device 1, users can be quickly and effectively aware of his/her current facial muscle status, so as to estimate whether the currently used care products/cosmetics and maintenance manners (such as working out or aesthetic medicine) are effective or not.

The analyzing device 1 shown in FIG. 1 and FIG. 2 is mainly used to assist the user in applying care products and cosmetics. More specific, the analyzing device 1 may provide makeup suggestions before the user start to apply cosmetics, provides makeup assistance while the user is making up, and provides analysis and evaluation for user's makeup after the user finishes applying cosmetics. It should be mentioned that, if other electronic devices, such as smart phones, laptops, etc., have same or similar hardware as the analyzing device 1 and are installed with corresponding software for performing each control step of the marking method of the present invention, the marking method of the present invention, therefore, can no longer be limited running on the analyzing device 1 of FIG. 1 and FIG. 2.

As shown in FIG. 1 and FIG. 2, the analyzing device 1 includes a processor 10, a display unit 11, an image capturing unit 12, an input unit 13, a wireless transmitting unit 14, and a storage 15.

The processor 10 is electrically connected with the display unit 11, the image capturing unit 12, the input unit 13, the wireless transmitting unit 14, and the storage 15 through serial bus, so as to integrate and control all of these components.

The analyzing device 1 can capture user's photo in real-time through the image capturing unit 12, then retrieves user's facial image from the photo and displays the facial image on the display unit 11. In one of the embodiments, the image capturing unit 12 is basically utilized to capture a photo including user's face. One of the plurality of technical features of the present invention is that the analyzing device 1 can identify the retrieved facial image and automatically marks a plurality of muscle feature points on the facial image for indicating user's current facial muscle status, and can also display simultaneously the facial image as well as the plurality of muscle feature points on the display unit 11. In a conclusion, user can be quickly aware of his/her current muscle status through the information displayed on the analyzing device 1.

The input unit 13 is arranged at one side of the analyzing device 1, and can be a physical style unit or a touch style unit. By using the input unit 13, the user is allowed to interact with the analyzing device 1, so as to operate the analyzing device and instruct the same. For example, the user can select different functions on the analyzing device 1 through operating the input unit 13 (such as a makeup assistance function, a facial muscle analysis function, etc.), or switch the makeup steps/makeup suggestions provided by the analyzing device 1 (such as page-up, page-down, etc.).

In one embodiment, the display unit 11 is a touch screen which can be operated by the user. In this embodiment, the input unit 13 and the display unit 11 are integrated into one component and not individually existed.

The wireless transmitting unit 14 is utilized to connect to the Internet, so the analyzing device 1 can connect to a remote electronic device or server through the Internet. In the present invention, the analyzing device 1 utilizes one or more algorithm to perform a training procedure for training an artificial intelligent (AI) model, a recognition procedure for recognizing the facial image, and a marking procedure for marking the muscle feature points. These algorithms and the AI model trained can be stored in the analyzing device 1 or the remote electronic device and server, not limited thereto. In addition, the user can operate a user terminal (not shown) to connect to the analyzing device 1 through network, so as to perform firmware maintenance and firmware updating to the analyzing device 1 from a remote place.

In one embodiment, the analyzing device 1 utilizes the image capturing unit 12 to capture user's facial image in real-time, and identifies the facial image to analyze user's current facial muscle status. In other embodiment, the analyzing device 1 downloads user's photo from a remote electronic device or server, and identifies the facial image included in the download photo for evaluating the facial muscle status of the user by the time the user took this photo. In another embodiment, the analyzing device 1 reads the user's photo from the storage 15, and identifies the facial image included in the photo for evaluating the facial muscle status of the user by the time the user took this photo.

The storage 15 stores the algorithms and the model(s) utilized to perform the marking method of the present invention. In particular, the storage 15 stores at least a training model 153 pre-trained for performing a fuzzy comparison procedure, and an AI training algorithm 151 as well as a bunch of training data 152 for training the training model 153, but not limited thereto. The AI training algorithm 151, the training data 152 as well as the training model 153 can also be stored in the remote electronic device or server, and the analyzing device 1 can connect to the remote electronic device or server through network for accessing the AI training algorithm 151, the training data 152, and the training model 153 from a remote place.

In another embodiment, the AI training algorithm 151 can be embedded in the processor 10 for being a part of the firmware of the processor 10, but not limited thereto.

In the present invention, the manufacturer of the analyzing device 1 can pre-import a bunch of training data 152 into the storage 15 of the analyzing device 1. The multiple records of training data 152 here indicate facial images of unspecified persons, and each of the facial images has been manually marked thereon with a plurality of muscle feature points defined by professionals such as doctors, cosmetologists, etc.... Therefore, the analyzing device 1 can perform a training procedure to train the training model 153 through executing the AI training algorithm 151 based on the multiple records of the training data 152. In this invention, the locations of the plurality of muscle feature points distributed on each of the facial images can be used to represent the muscle status of the face.

One of the technical features of the present invention is that, when obtaining a new photo (a facial image captured by the image capturing unit 12 or a photo read from the storage 15), the analyzing device 1 can perform a fuzzy comparison procedure to the photo based on the training model 153, and automatically marks a plurality of muscle feature points on a facial image included in the photo according to a result of the fuzzy comparison procedure. Therefore, the user can determine his/her current facial muscle status according to the locations of the plurality of muscle feature points on the facial image automatically marked by the analyzing device 1.

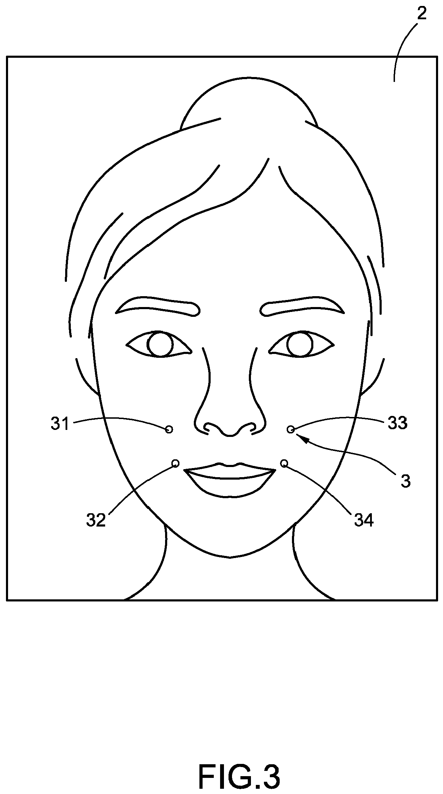

FIG. 3 is a schematic diagram showing muscle feature points according to a first embodiment of the present invention. As disclosed in FIG. 3, after obtaining a to-be-identified image 2 which includes user's facial image through the image capturing unit 12, the wireless transmitting unit 14, or the storage 15, the analyzing device 1 can perform the fuzzy comparison procedure to the to-be-identified image 2 according to the pre-trained training model 153 for finding the locations for a plurality of muscle feature points 3 distributed on the facial image of the to-be-identified image 2, so as to mark the plurality of muscle feature points 3 on the facial image of the to-be-identified image 2 and then displays the muscle feature points 3 as well as the to-be-identified image 2 on the display unit 11.

In the embodiment shown in FIG. 3, the analyzing device 1 automatically marks at least four muscle feature points 3 on the to-be-identified image 2 according to the training model 153, the at least four muscle feature points 3 includes a first muscle feature point 31 located at upper-left of the face (corresponding to a location of left risorius muscle), a second muscle feature point 32 located at lower-left of the face (corresponding to a location of left masticatory muscle), a third muscle feature point 33 located at upper-right of the face (corresponding to a location of right risorius muscle), and a fourth muscle feature point 34 located at lower-right of the face (corresponding to a location of right masticatory muscle). The amount of the plurality of muscle feature points 3 illustrated in FIG. 3 is four, however, it can be more than four in other embodiments.

It should be mentioned that within a certain range, the user's muscle status is regarded great once the first muscle feature point 31 as well as the third muscle feature point 33 are close to inner side and upper side of the face (i.e., close to nose and eyes). Similarly, within a certain range, the user's muscle status is regarded great once the second muscle feature point 32 as well as the fourth muscle feature point 34 are close to inner side and upper side of the face (i.e., close to mouth and nose).

As mentioned above, the marking method of the present invention is to identify the to-be-identified image 2 through the pre-trained training model 153, and automatically marks at least four muscle feature points 3 on the to-be-identified image 2 after the identification procedure. Therefore, a technical effect can be achieved that the user can check his/her current muscle status according to the locations of the at least four muscle feature points 3 marked.

In comparison with multiple strong reference points on a face (such as organs like eyes, nose, mouth, or facial parts with obvious characteristics), the muscle feature points 3 defined in the present invention are mainly corresponding to multiple weak reference points on the face. As a result, before utilizing the analyzing device 1 to implement the marking method of the present invention which automatically marks such muscle feature points 3 on a to-be-identified image 2 input by the user, the training model 153 needs to be pre-established for the analyzing device 1 to perform such fuzzy comparison procedure.

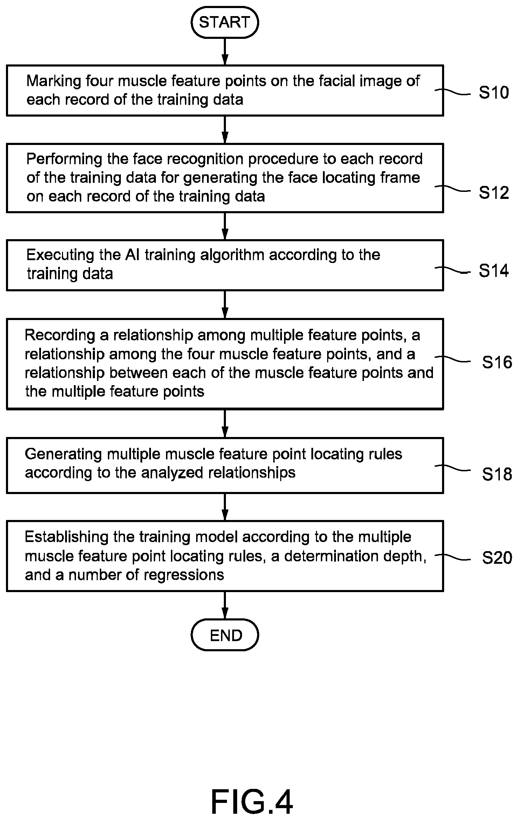

Please refer to FIG. 4, which is a flowchart for establishing a training model according to a first embodiment of the present invention, and is illustrated to specifically describe an establishing procedure of the training model 153 of the present invention.

As shown in FIG. 4, firstly, the user obtains a bunch of records of the training data 152, these records of the training data 152 respectively indicates different facial image of different people.

Next, the user manually marks at least four muscle feature points on each of the facial image of each record of the training data 152 according to the facial muscle status represented by each facial image based on one or more feature point setting rules defined by professionals such as doctors or cosmetologist (step S10). As discussed before, the present invention utilizes the at least four muscle feature points to represent the muscle status of a face, in other words, in a previous training phase the user can manually mark multiple muscle feature points on each of the records of the training data 152 according to the real muscle status represented by each face in each of the records of the training data 152, and regards these marked records of training data 152 as a training foundation when establishing the training model 153.

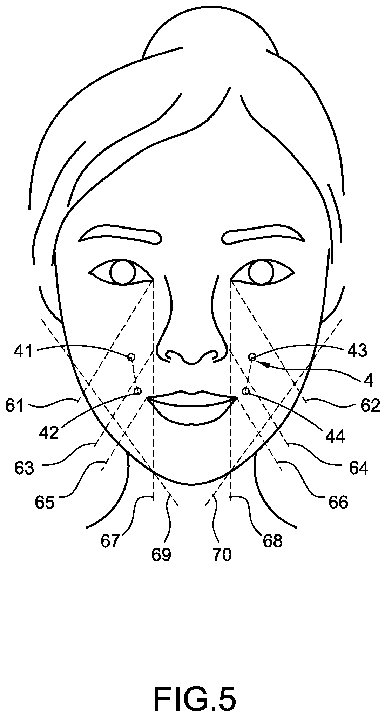

FIG. 5 is a schematic diagram showing the marked location of the muscle feature points according to a first embodiment of the present invention. In the present invention, the manually marked muscle feature points 4 includes a first muscle feature point 41 and a second muscle feature point 42 located at left side of a face, and includes a third muscle feature point 43 and a fourth muscle feature point 44 located at right side of the face. In the embodiment, the at least four muscle feature points 4 are respectively located at four weak reference points of the face, in other words, an amount of the weak reference points is corresponding to an amount of the muscle feature points 4 marked. In particular, even if the amount of the muscle feature points 4 the user marked on each facial image included in each record of the training data 152 is greater than four, each of the muscle feature points 4 should be still marked at a weak reference point of the face image of each record of the training data 152.

More specific, the user may perform analysis and determination to the facial image of each record of the training data 152 by bare eyes or algorithm, so as to obtain multiple area constituting assistance lines including a first tear trough tangent 61, a first nasolabial fold tangent 63, a first marionette line tangent 65, a first vertical line of eye corner 67, and a first mandible ramus tangent 69 on left side of the face as well as multiple area constituting assistance lines including a second tear trough tangent 62, a second nasolabial fold tangent 64, a second marionette line tangent 66, a second vertical line of eye corner 68, and a second mandible ramus tangent 70 on right side of the face according to the real muscle status represented by the face inside of each record of the training data 152. In the present invention, the user may decide specific locations for the muscle feature points 4 according to such area constituting assistance lines, and then directly and manually marks the at least four muscle feature points 4 on each record of the training data 152.

When marking such muscle feature points 4, the user should make the first muscle feature point 41 to be located and marked within a region constituted by the first tear through tangent 61, the first nasolabial fold tangent 63, the first vertical line of eye corner 67, and the first mandible ramus tangent 69 at the left side of the face; make the second muscle feature point 42 to be located and marked within a region constituted by the first nasolabial fold tangent 63, the first marionette line tangent 65, the first vertical line of eye corner 67, and the first mandible ramus tangent 69 at the left side of the face; make the third muscle feature point 43 to be located and marked within a region constituted by the second tear trough tangent 62, the second nasolabial fold tangent 64, the second vertical line of eye corner 68, and the second mandible ramus tangent 70 at the right side of the face; and make the fourth muscle feature point 44 to be located and marked within a region constituted by the second nasolabial fold tangent 64, the second marionette line tangent 66, the second vertical line of eye corner 68, and the second mandible ramus tangent 70 at the right side of the face. The above description is only one of the exemplary embodiments of the present invention, but not limited thereto.

In addition, the analyzing device 1 may determine if each of the muscle feature points 4 is marked within a corresponding region constituted by corresponding area constituting assistance lines, so as to pre-determine whether the muscle feature points 4 manually marked by the user are correct or not, and filters mis-marked muscle feature point(s) 4 or makes an alert for the user to double check. Similarly, the above assistance approach can also be applied in the following procedure after the training model 153 is well-trained, so as to optionally determine whether the muscle feature points 3 automatically marked according to the training model 153 are correct or not. By using such assistance approach, the marking method of the present invention can be effectively assisted when the amount of the records of the training data 152 is not sufficiently enough while the training model 153 is trained.

Please refer back to FIG. 4. The user initially performs a face recognition procedure to each record of the training data 152 through the analyzing device 1 or other electronic device, so as to find and locate multiple facial features from the record of the training data 152 for being the training foundations. In one embodiment, the analyzing device 1 executes a histogram of oriented gradient (HOG) algorithm of Dlib Face Landmark system by the processor 10 of the analyzing device 1 to perform the face recognition procedure to each record of the training data 152, and generates a face locating frame on each record of the training data 152 for indicating effective facial features thereon (step S12). In one of the exemplary embodiments, at least a part of the facial features are strong reference points of the face included in each record of the training data 152.

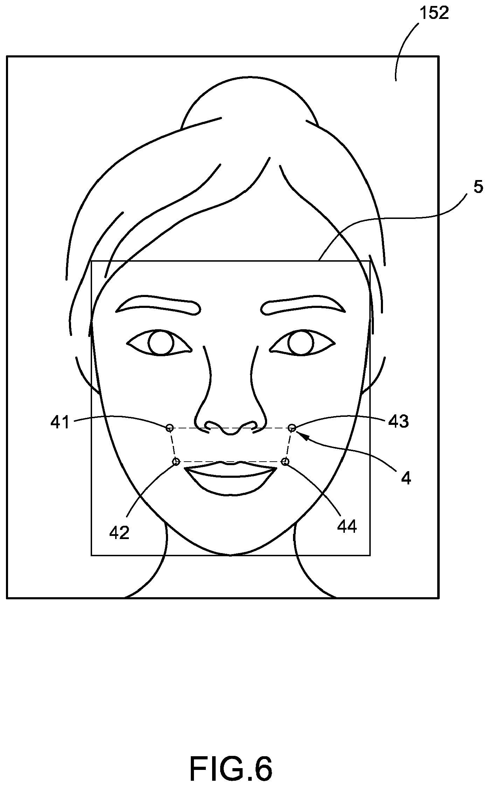

Please also refer to FIG. 6, which is a schematic diagram showing training data according to a first embodiment of the present invention. As disclosed in FIG. 6, the aforementioned HOG algorithm is executed to transform a photo into multiple vectors, and determines a location of a facial image in the photo according to the combination of the size, direction, shape, etc., of these vectors, and also generates a face locating frame 5 which can cover the whole facial image. In the embodiment, the fourth muscle feature points 4 are all located within the face locating frame 5, and the connection of the four muscle feature points 4 can virtually form a rectangle frame or a trapezoid frame.

Refer back to FIG. 4. After the step S12, the analyzing device 1 may establish the training model 153 through executing the AI training algorithm 151 according to the training data 152 (step S14). More specific, the AItraining algorithm 151 is executed to perform a training procedure based on the multiple records of the training data 152, the muscle feature points 4 manually marked on each record of the training data 152, the face locating frame 5 of each record of the training data 152, and the multiple facial features within the face locating frame 5 of each record of the training data 152.

It should be mentioned that the user may optionally choose to mark these muscle feature points 4 first, or to make the analyzing device 1 to generate the face locating frame 5 first. In other words, the step S10 and the step S12 do not have an essential executing order.

In the present invention, the AI training algorithm 151 is executed to analyze the training data 152 during the training procedure, and records at least a relationship among multiple feature points within the face locating frame 5, a relationship among each of the plurality of muscle feature points 4 (such as the size, the shape, the angles of the rectangle frame or the trapezoid shape), a relationship between each muscle feature point 4 and the one or more feature points (especially strong reference points) within the face locating frame 5, etc. (step S16).

It is worth saying that the AI training algorithm 151, in the step S16, can also calculate the probability of each muscle feature point 4 appearing at each location of the face (for example, where are the locations that these muscle feature points 4 are never located) during the training procedure, and also generates a basic locating rule of each of the muscle feature points 4 (for example, the location of the first muscle feature point 41 is must higher than the location of the second muscle feature point 42, the third muscle feature point 43 is must located at a right side of the first muscle feature point 41, etc.), and the probability and the basic locating rule can be utilized by the analyzing device 1 as predicted reference values while performing the fuzzy comparison procedure to a to-be-identified image 2 as newly provided.

After the step S16, the AI training algorithm 151 can generate multiple muscle feature point locating rules according to the corresponding relationships obtained by the analysis performed in the step S16 (step S18), and then establishes a training model 153 according to the multiple muscle feature point locating rules, a determination depth, and a number of regressions (step S20). After the training model 153 is established, the analyzing device 1 can perform the fuzzy comparison procedure to a to-be-identified image newly inputted, so as to automatically mark the aforementioned multiple muscle feature points 3 on a facial image in the to-be-identified image 2 based on the training model 153.

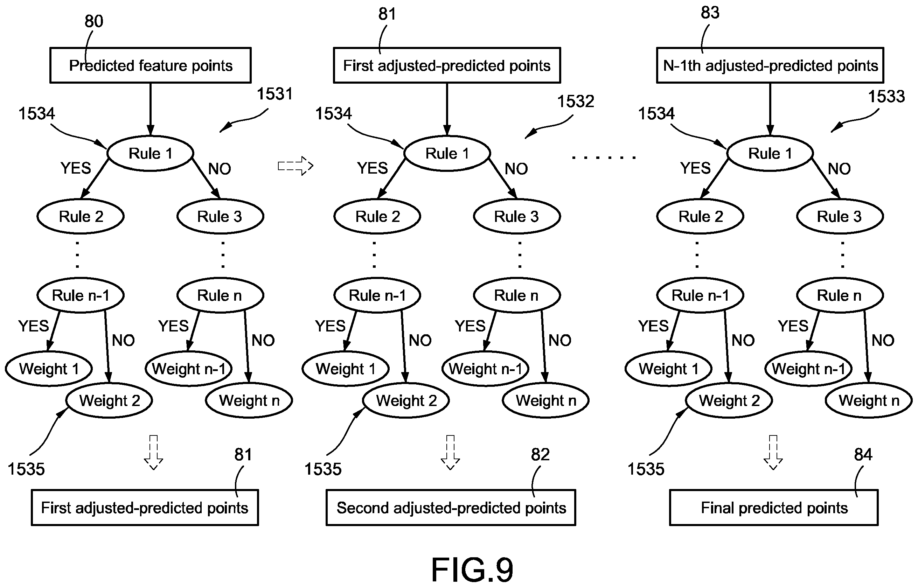

In the present invention, the training model 153 generated by the AI training algorithm 151 is a type of regressor, which includes multiple Cascade regression trees with same contents. Each of the regression trees included in the regressor (i.e., in the training model 153) respectively has multiple determination nodes, and the contents of at least a part of the determination nodes are corresponding to the above muscle feature point locating rules (as shown in FIG. 9, detailed described below).

It should be mentioned that, before the AI training algorithm 151 is executed, the user may preset the number of the regressions and the number of the determination nodes, according to parameters such as hardware performance of the analyzing device 1, required accuracy of the identification result, processing time acceptable to the user, etc.. In the present embodiment, the number of the regression trees equals the pre-set number of regressions, and the pre-set number of determination nodes equals the determination depth. Without concerning the hardware performance, accuracy of the identification result and the processing time, the identification result will be more accurate once the number of regressions and the determination depth are bigger.

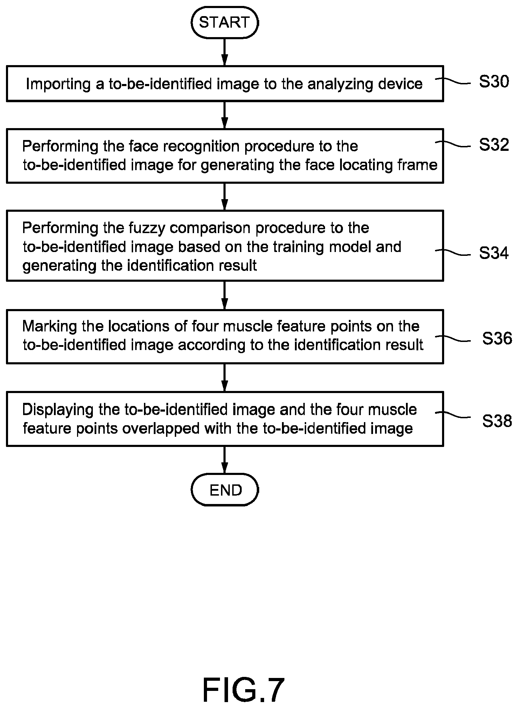

Please refer to FIG. 7, which is an identification flowchart according to a first embodiment of the present invention. FIG. 7 discloses each execution step of the marking method of the present invention, where the marking method is applied to the analyzing device 1 as shown in FIG. 1 and FIG. 2.

When using the analyzing device 1 to identify the muscle status of his/her face, the user imports a to-be-identified image 2 (as shown in FIG. 3) having user's facial image to the analyzing device 1, and the analyzing device 1 can obtain the to-be-identified image 2 of the user (step S30). In one embodiment, the analyzing device 1 may real-time capture the to-be-identified image 2 through its image capturing unit 12. In other embodiment, the analyzing device 1 can read the to-be-identified image 2 pre-stored by the user from the storage 15. In another embodiment, the analyzing device 1 may wirelessly receive the to-be-identified image 2 from the outside through the wireless transmitting unit 14.

After obtaining the to-be-identified image 2, the analyzing device 1 performs the face recognition procedure to the to-be-identified image 2 through the processor 10, and the face locating frame 5 as shown in FIG. 6 is then generated according to the result of the face recognition procedure (step S32). The face locating frame 5 indicates a certain facial image included in the to-be-identified image 2, in particular, the face locating frame 5 covers multiple strong reference points, such as organs like eyes, nose, and mouth, or obvious characteristic points, upon the facial image included in the to-be-identified image 2.

In one embodiment, the processor 10 performs the face recognition procedure to the to-be-identified image 2 through the HOG algorithm of Dlib Face Landmark system as mentioned above, and generate such face locating frame 5 on the to-be-identified image 2 for indicating the facial image of the user. In the present invention, the processor 10 only perform training for the training model 153 according to multiple feature points within the face locating frame 5 of each record of the training data 152. As a result, when performing the identification procedure, the processor 10 will only perform identification according to multiple feature points within the face locating frame 5 of the to-be-identified image 2.

After the step S32, the processor 10 performs fuzzy comparison procedure to the to-be-identified image 2 based on the training model 153 and then generates an identification result (step S34). Therefore, the processor 10 can mark the locations of four muscle feature points 3 on the to-be-identified image 2 according to the identification result (step S36). In the present invention, the four muscle feature points 3 will be located within the face locating frame 5 of the to-be-identified image 2 and respectively corresponded to at least four weak reference points upon use's facial image in the to-be-identified image 2.

After the step S36, the processor 10 controls the display unit 11 to display the to-be-identified image 2 as well as the four muscle feature points 3 (step S38), and the four muscle feature points 3 are respectively overlapped with a corresponding location on the facial image in the to-be-identified image 2.

In the marking method of the present invention, the processor 10 performs the fuzzy comparison procedure to the to-be-identified image 2 based on the training model 153, so the locations of the four muscle feature points 3 automatically marked by the processor 10 on the to-be-identified image 2 will be complying with the locating rules pre-analyzed according to the bunch of records of the training data 152.

In particular, an amount of the multiple muscle feature points 3 automatically marked on the to-be-identified image 2 through the fuzzy comparison procedure is corresponding to an amount of the multiple muscle feature points 4 manually marked in each of the records of the training data 152. In other words, the amount of the muscle feature points 3 marked by the processor 10 in step S36 and the amount of the muscle feature points 3 displayed on the to-be-identified image 2 in the step S38, are corresponding to the amount of the muscle feature points 4 marked in each of the records of the training data 152 stored in the storage 15, i.e., the amount is plurality, but not limited at four.

In one embodiment, the four muscle feature points 3 automatically marked by the processor 10 includes a first muscle feature point 31 and a second muscle feature point 32 located at a left side of the facial image in the to-be-identified image 2, and also includes a third muscle feature point 33 and a fourth muscle feature point 34 located at a right side of the facial image in the to-be-identified image 2. In an embodiment, the connection of the four muscle feature points 3 can virtually form a rectangle frame or a trapezoid frame.

In other embodiment, the location of the first muscle feature point 31 automatically marked by the processor 10 will be within a region constituted by the aforementioned first tear trough tangent 61, the first nasolabial fold tangent 63, the first vertical line of eye corner 67, and the first mandible ramus tangent 69 on a left side of the facial image; the location of the second muscle feature point 32 will be within a region constituted by the first nasolabial fold tangent 63, the first marionette line tangent 65, the first vertical line of eye corner 67, and the first mandible ramus tangent 69 at the left side of the facial image; the location of the third muscle feature point 33 will be within a region constituted by the second tear trough tangent 62, the second nasolabial fold tangent 64, the second vertical line of eye corner 68, and the second mandible ramus tangent 70 at a right side of the facial image; and the location of the fourth muscle feature point 34 will be within a region constituted by the second nasolabial fold tangent 64, the second marionette line tangent 66, the second vertical line of eye corner 68, and the second mandible ramus tangent 70 at the right side of the facial image.

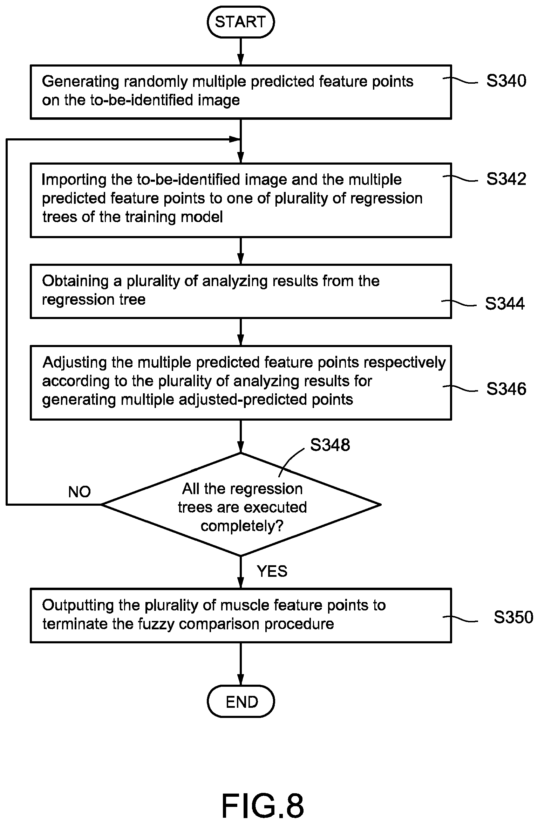

As mentioned above, the present invention provides the analyzing device 1 to automatically mark at least four muscle feature points 3, so the user can quickly and effectively check his/her current facial muscle status. For example, if the locations of the first muscle feature point 31 and the third muscle feature point 33 automatically marked are close to the eyes of user's facial image, it means user's muscle is very tight. For another example, if the locations of the second muscle feature point 32 and the fourth muscle feature point 34 automatically marked are far from the mouth and close to the chin of user's facial image, it means the user's muscle is flabby and needed to be maintained. Please refer to FIG. 8, which is an identification flowchart according to a second embodiment of the present invention, and is used to detailed describe the specific content of the step S34 as disclosed in FIG. 7.

As mentioned above, the training model 153 is a type of regressor which includes multiple Cascade regression trees. As also mentioned before, the AI training algorithm 151 may analyze the bunch of records of the training data 152 and record the probability of each muscle feature point appearing at each location and the basic locating rule of each of the muscle feature points during establishing the training model 153. Before performing the fuzzy comparison procedure to the to-be-identified image 2 based on the training model 153, the processor 10 first randomly generates multiple predicted feature points on the to-be-identified image 2 according to the probability and the basic locating rule (step S340). In this embodiment, the multiple predicted feature points are randomly generated and regarded as an initial estimate. The initial estimate can exclude the locations which are impossible for the muscle feature points (e.g., upper of the eyebrow, inside of the mouth, etc.) according to the probability, and ensures the multiple predicted feature points generated are all complied with the basic locating rule (for example, the multiple predicted feature points are all located within the face locating frame 5, the connection of the multiple predicted feature points can virtually form a rectangle frame or a trapezoid frame, etc.).

It is worth saying that the amount of the multiple predicted feature points is corresponding to the amount of the multiple muscle feature points 4 manually marked in each of the records of the training data 152. In other words, the amount of the multiple predicted feature points randomly generated by the processor 10 in the step S340 equals the amount of the multiple muscle feature points 4 respectively marked in each of the records of the training data 152 which is used to train the training model 153. The number is plurality, but not limited at four.

Next, the processor 10 imports the to-be-identified image 2 and the multiple predicted feature points to a selected one of plurality of regression trees of the training model 153 (step S342), and obtains a plurality of analyzing results from the selected regression tree (step S344). After the step S344, the processor 10 adjusts the multiple predicted feature points respectively according to the plurality of analyzing results obtained from the selected regression tree for generating multiple adjusted-predicted points (step S346).

In the present invention, each of the plurality of analyzing results respectively indicates a weight of the combination of the to-be-identified image 2 incorporated with the multiple predicted feature points in comparison with the content of at least a part of the multiple records of the training data 152.

In particular, each weight is utilized to indicate a similarity of a relationship between one or more strong reference points with each of the predicted feature points in the to-be-identified image 2 in comparison with a relationship between one or more strong reference points and each of the muscle feature points 4 in a record of the training data 152. In other words, the analyzing results can be used to indicate the similarity of a combination of the to-be-identified image 2 incorporated with the current predicted feature points thereon in comparison with a combination of each record of the training data 152 (or the induced/categorized data cluster of the training data 152) incorporated with the muscle feature points 4 thereon.

In one embodiment, the higher the similarity, the bigger the weight is. In the step S346, the processor 10 respectively adjusts the coordinates of each predicted feature point on the to-be-identified image 2 according to different weights (i.e., the plurality of analyzing results), so as to generate a plurality of adjusted-predicted points. The amount of the plurality of adjusted-predicted points equals the amount of the plurality of predicted feature points (such as four in this embodiment).

As mentioned above, the training model 153 includes a plurality of regression trees, and the amount of the regression trees is corresponding to the number of regressions preset by the user. After the step S346, the processor 10 determines whether the plurality of regression trees of the training model 153 are all executed completely (step S348), i.e., the processor 10 determines if the execution time of the step S342, step S344, and step S346 are all equal to the number of regressions preset by the user.

In the present invention, before the plurality of regression trees is all executed completely, the processor 10 replaces the previous plurality of predicted feature points with the plurality of adjusted-predicted points generated in the step S346, and re-executes the step S342, the step S344, and the step S346 in next regression tree of the plurality of regression trees according to the to-be-identified image 2 and the plurality of adjusted-predicted points. When the plurality of regression trees is all executed completely, the processor 10 regards the plurality of adjusted-predicted points generated in the last step S346 as a plurality of final-confirmed muscle feature points 3, and the processor 10 outputs the plurality of muscle feature points 3 to terminate the fuzzy comparison procedure (step S350). In the present invention, the multiple muscle feature points 3 automatically marked on the to-be-identified image 2 by the analyzing device 1 are the plurality of final-confirmed muscle feature points 3 output by the processor 10 in the step S350.

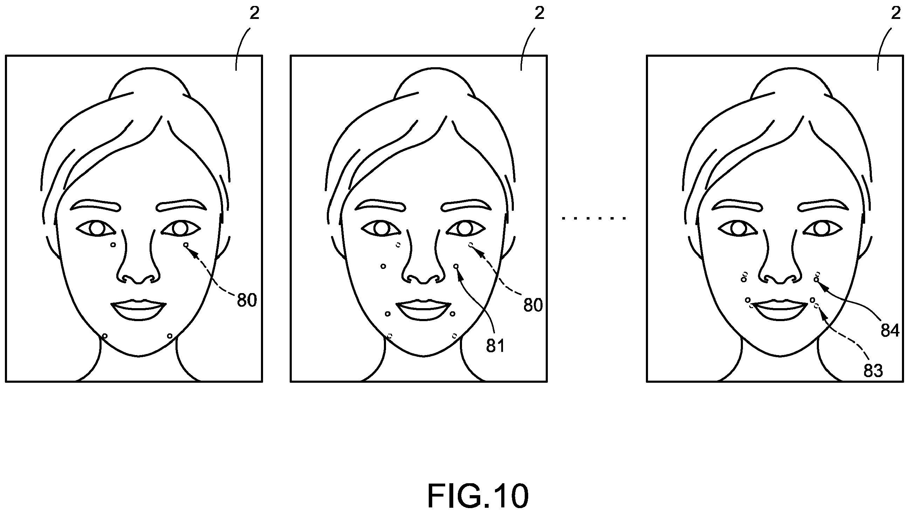

Please refer to FIG. 9 and FIG. 10, wherein FIG. 9 is a schematic diagram showing a training model according to a first embodiment of the present invention, FIG. 10 is a schematic diagram showing the adjustment of the feature points according to a first embodiment of the present invention. For easily understanding, four predicted feature points will be illustrated in FIG. 9 and FIG. 10 for an example (i.e., four muscle feature points 4 are manually marked in each of the records of the training data 152).

As shown in the FIGs, before performing the fuzzy comparison procedure, the processor 10 randomly generates four predicted feature points 80 on the to-be-identified image 2 according to the basic locating rule and the probability as described before. It should be noticed that these four predicted feature points 80 merely comply with the basic locating rule and the probability calculated by the AI training algorithm 151 during the training procedure, but cannot represent the actual muscle status of the facial image included in the to-be-identified image 2.

Next, the processor 10 imports the to-be-identified image 2 and the four predicted feature points 80 into a first regression tree 1531 of the plurality of regression trees of the training model 153. The first regression tree 1531 has multiple determination nodes 1534, each of the multiple determination nodes 1534 respectively represents one of a plurality of rules, these rules are induced, calculated and obtained by the AItraining algorithm 151 during training the training model 153 based on the plurality of records of training data 152.

In the present invention, the content of at least a part of the determination nodes 1534 of the first regression tree 1531 are corresponding to the multiple muscle feature point locating rules generated by the AI training algorithm 151 in the step S18 as shown in FIG. 4, in other words, the processor 10 will determine, at each of the determination nodes 1534, whether the relationship among each of the four predicted feature points 80 on the to-be-identified image 2 (e.g., the size or the shape of the virtual rectangle frame or a trapezoid frame) is similar to the rule indicated by the content of each of the determination nodes 1534, or whether the relationship between each of the predicted feature points 80 on the to-be-identified image 2 and the one or more strong reference points within the face locating frame 5 is similar to the rule indicated by the content of each of the determination nodes 1534. That is to say, the processor 10 determines a YES answer (i.e., similar) or a NO answer (i.e., dissimilar) at each of the determination nodes 1534.

When determining YES or NO at each determination node 1534, the processor 10 is to basically determine a similar probability and a dissimilar probability, but neither the similar probability nor the dissimilar probability will be 100%. If the plurality of determination nodes 1534 included in the regression tree 1534 are all determined completely, the processor 10 can obtain multiple analyzing results from the plurality of determination nodes 1534, and each of the multiple analyzing results is respectively corresponding to one of the multiple weights 1535.

In the present invention, each of the obtained weights 1535 is respectively used to indicate the similarity of the relationship between each of the predicted feature points 80 and the one or more strong reference points on the facial image of the to-be-identified image 2 and the relationship between each of the muscle feature points 4 and the one or more strong reference points in each of the records of the training data 152 (or a cluster of multiple similar records of the training data 152), or, each of the weights 1535 is also used to respectively indicate the similarity of the relationship among each of the predicted feature points 80 on the facial image of the to-be-identified image 2 and the relationship among each of the muscle feature points 4 in each of the records of the training data 152 (or such cluster).

For example, if the facial image in the to-be-identified image 2 is similar to a first type of record of the training data 152 (e.g., indicating a face with a big nose), the corresponding weight 1535 will be higher. If the facial image in the to-be-identified image 2 is dissimilar to a second type of record of the training data 152 (e.g., indicating a face with small eyes), the corresponding weight 1535 will be lower.

After all the determination nodes 1534 in the first regression tree 1531 are determined completely and multiple weights 1535 are obtained, the processor 10 further adjusts the coordinates of each of the predicted feature points 80 upon the to-be-identified image 2 according to these weights 1535, so as to generate a plurality of first adjusted-predicted points 81. For example, if the facial image of the to-be-identified image 2 is similar to a first type of record of the training data 152 (e.g., indicating a face with a big nose), the coordinates of the first adjusted-predicted points 81 will be adjusted slightly to move toward the locations of the multiple muscle feature points 4 marked in the first type of record of the training data 152, and the adjusting range of the coordinates is corresponding to the content of the weight 1535 (i.e., the higher the weight 1535, the bigger the adjusting range will be).

As disclosed in FIG. 10, the first adjusted-predicted points 81 are closer to the actual locations of the multiple muscle feature points 3 on the facial image of the to-be-identified image 2 in comparison with the predicted feature points 80, wherein, the amount of the plurality of first adjusted-predicted points 81 equals the amount of the plurality of predicted feature points 80.

Refer back to FIG. 9. After obtaining the four first adjusted-predicted points 81, the processor 10 further imports the to-be-identified image 2 and the four first adjusted-predicted points 81 into a second regression tree 1532 of the training model 153. In the present invention, the content of the second regression tree 1532 is identical with the content of the aforementioned first regression tree 1531. Also, the second regression tree 1532 includes multiple determination nodes 1534, the amount and content of the multiple determination nodes 1534 in the second regression tree 1532 are identical to the amount and content of the multiple determination nodes 1534 in the first regression tree 1531. However, the imported parameters are changed, from the four predicted feature points 80 to the four first adjusted-predicted points 81, so the plurality of analyzing results will be different after the second regression tree 1532 is executed completely (i.e., the content of the multiple weights 1535 obtained from the second regression tree 1532 will be different).

Similarly, after all the determination nodes 1534 in the second regression tree 1532 are determined completely and multiple weights 1535 are obtained, the processor 10 further adjusts the coordinates of each of the first adjusted-predicted points 81 upon the to-be-identified image 2 according to the obtained weights 1535, so as to generate multiple second adjusted-predicted points 82.

In the invention, the processor 10 keeps executing above actions (regressions and adjustments). After an N-1 regression tree (not shown) of the training model 153 is executed completely, the processor 10 generates four N-1th adjusted-predicted points 83. Next, the processor 10 imports the to-be-identified image 2 and the four N-1th adjusted-predicted points 83 into a last regression tree of the training model 153 (an Nth regression tree 1533 in FIG. 9). After the Nth regression tree 1533 is executed completely, the processor 10 can obtain multiple analyzing results (i.e., multiple weights 1535), and the processor 10 can then adjust the four N-1th adjusted-predicted points 83 according to the multiple weight 1535 obtained from the Nth regression tree 1533, so as to generate four final predicted points 84. In this invention, the processor 10 will outputs these four final predicted points 84 as the four muscle feature points 3 automatically analyzed by the analyzing device 1, and automatically marks the four muscle feature points 3 on the to-be-identified image 2.

It is worth to say that the number of the regression trees is decided depending on hardware performance of the analyzing device 1, identification accuracy required by the user, and processing time acceptable to the user, etc... In general, once the number of regression trees gets more, the locations of the final predicted points 84 will be way closer to the correct locations that the actual muscle feature points should be on the facial image of the to-be-identified image 2.

By using the marking method of the present invention, the analyzing device 1 can automatically mark at least four muscle feature points on user's facial image by way of AI function, so the user may quickly and effectively be aware of his/her current facial muscle status, and determines whether the care products/cosmetics/maintenance manner currently applied is working or not. As a conclusion, the technical solution provided by the present invention is convenient and useful to the user.

## Claims

1. A method for automatically marking muscle feature points on face, applied to a facial image analyzing device (1) having a processor (10), a display unit (11), and a storage (15) stored with a pre-trained training model (153), comprising:
a) obtaining a to-be-identified image (2) of a user;
b) performing a face recognition procedure to the to-be-identified image (2) by the processor (10) for generating a face locating frame (5), wherein the face locating frame (5) indicates a face in the to-be-identified image (2) and covers multiple strong reference points of the face;
c) performing a fuzzy comparison procedure to the to-be-identified image (2) based on the training model (153) by the processor (10) for generating an identification result;
d) marking multiple muscle feature points (3) on the face of the to-be-identified image (2) according to the identification result, wherein the multiple muscle feature points (3) are located within the face locating frame (5) and corresponding to multiple weak reference points of the face; and
e) displaying the to-be-identified image (2) and displaying the multiple muscle feature points (3) overlapped with the face of the to-be-identified image (2) by the display unit (11).

2. The method for automatically marking muscle feature points on face in claim 1, wherein the multiple muscle feature points (3) comprises at least four muscle feature points, the at least four muscle feature points comprises a first muscle feature point (31) and a second muscle feature point (32) located at a left side of the face, and comprises a third muscle feature point (33) and a fourth muscle feature point (34) located at a right side of the face, wherein a connection of the four muscle feature points virtually forms a rectangle frame or a trapezoid frame.

3. The method for automatically marking muscle feature points on face in claim 2, wherein the first muscle feature point (31) is located within a region constituted by a first tear through tangent (61), a first nasolabial fold tangent (63), a first vertical line of eye corner (67), and a first mandible ramus tangent (69) at the left side of the face, and the third muscle feature point (33) is located within a region constituted by a second tear trough tangent (62), a second nasolabial fold tangent (64), a second vertical line of eye corner (68), and a second mandible ramus tangent (70) at the right side of the face.

4. The method for automatically marking muscle feature points on face in claim 3, wherein the second muscle feature point (32) is located within a region constituted by the first nasolabial fold tangent (63), a first marionette line tangent (65), the first vertical line of eye corner (67), and the first mandible ramus tangent (69) at the left side of the face, the fourth muscle feature point (34) is located within a region constituted by the second nasolabial fold tangent (64), a second marionette line tangent (66), the second vertical line of eye corner (68), and the second mandible ramus tangent (70) at the right side of the face.

5. The method for automatically marking muscle feature points on face in claim 1, wherein the step a) is to real-time capture the to-be-identified image (2) by an image capturing unit (12) of the facial image analyzing device (1), read the to-be-identified image (2) from the storage (15) or externally receive the to-be-identified image (2) through a wireless transmitting unit (14) of the facial image analyzing device (1), wherein the to-be-identified image (2) comprises at least a facial image of the face of the user.

6. The method for automatically marking muscle feature points on face in claim 1, wherein the step b) is to perform the face recognition procedure to the to-be-identified image (2) for generating the face locating frame (5) through a Histogram of Oriented Gradient (HOG) algorithm of Dlib Face Landmark system.

7. The method for automatically marking muscle feature points on face in claim 1, further comprising following steps before the step a):
a01) marking the multiple muscle feature points (4) respectively on each of a plurality of records of training data (152), wherein each of the records of the training data (152) respectively comprises a facial image;
a02) performing the face recognition procedure to each of the records of the training data (152) through a Histogram of Oriented Gradient (HOG) algorithm of Dlib Face Landmark system for generating the face locating frame (5) respectively on each of the records of the training data (152);
a03) executing an Artificial Intelligent (AI) training algorithm (151) according to the training data (152) for analyzing and recording a relationship among the multiple muscle feature points (4) of each of the records of the training data (152) and a relationship between each of the muscle feature points (4) and one or more strong reference points within the face locating frame (5) of each of the records of the training data (152);
a04) generating multiple muscle feature point locating rules according to the relationships analyzed
and recorded in the step a03); and
a05) establishing the training model (153) according to at least the multiple muscle feature point locating rules, a determination depth, and a number of regressions.

8. The method for automatically marking muscle feature points on face in claim 7, wherein the training model (153) is a regressor, the regressor comprises multiple Cascade regression trees having same contents, each of the regression trees (1531,1532,1533) respectively comprises multiple determination nodes (1534), at least a part of the multiple determination nodes (1534) are corresponding to the multiple muscle feature point locating rules, wherein an amount of the multiple regression trees (1531,1532,1533) equals the number of regressions, and an amount of the multiple determination nodes (1534) equals the determination depth.

9. The method for automatically marking muscle feature points on face in claim 8, wherein the step c) comprises following steps of:
c01) randomly generating multiple predicted feature points (80) corresponded to the multiple muscle feature points (3) on the to-be-identified image (2) according to a basic locating rule and a probability indicated by the training model (153);
c02) importing the to-be-identified image (2) and the multiple predicted feature points (80) into one of the multiple regression trees (1531, 1532, 1533) of the training model (153);
c03) obtaining multiple analyzing results of the regression tree (1531, 1532, 1533) used in the step c02);
c04) adjusting the multiple predicted feature points (80) according to the multiple analyzing results for generating multiple adjusted-predicted points (81, 82, 83);
c05) re-executing the step c02) to the step c04) according to the multiple adjusted-predicted points (81, 82, 83) before all of the multiple regression trees (1531, 1532, 1533) of the training model (153) are executed completely; and
c06) regarding the multiple adjusted-predicted points (81,82,83) as the multiple muscle feature points (3) of the to-be-identified image (2) after all of the multiple regression trees (1531,1532,1533) are executed completely.

10. The method for automatically marking muscle feature points on face in claim 9, wherein each of the multiple analyzing results respectively records a weight (1535) of the to-be-identified image (2) incorporated with the multiple predicted feature points (80) in comparison with the content of at least a part of the multiple records of the training data (152), the weight (1535) indicates a similarity of a relationship between one or more strong reference points and each of the predicted feature points (80) on the to-be-identified image (2) and a relationship between one or more strong reference points and each of the multiple muscle feature points (4) on each of the records of the training data (152), wherein the step c04) is to adjust a coordinates of each of the predicted feature points (80) upon the to-be-identified image (2) according to multiple weights (1535).

11. The method for automatically marking muscle feature points on face in claim 1, wherein the processor (10) recognizes a plurality of area constituting assistance lines on the face within the face locating frame (5), the plurality of area constituting assistance lines comprises a first tear through tangent (61), a first nasolabial fold tangent (63), a first marionette line tangent (65), a first vertical line of eye corner (67), and a first mandible ramus tangent (69) at the left side of the face, and also comprises a second tear trough tangent (62), a second nasolabial fold tangent (64), a second marionette line tangent (66), a second vertical line of eye corner (68), and a second mandible ramus tangent (70) at the right side of the face., wherein the processor (10) determines whether the multiple muscle feature points (3) are respectively located within each corresponding region constituted by the plurality of area constituting assistance lines, and determines that the identification result is incorrect if any one of the muscle feature points (3) does not locate within the corresponding region.
